# EUROPÄISCHE PATENTSCHRIFT

(11) **EP 1 301 788 B1**
(45) Veröffentlichungstag und Bekanntmachung des Hinweises auf die Patenterteilung: **28.01.2004**
(21) Anmeldenummer: 01965075.3
(22) Anmeldetag: 06.07.2001
(51) Int. Cl.: G01N 33/52, C12Q 1/24, G01N 33/569, B01L 3/00, C12M 1/30, B01L 11/00

(54) **VERFAHREN ZUR DURCHFÜHRUNG EINER SPEICHELANALYSE**
METHOD FOR CARRYING OUT A SALIVA ANALYSIS
PROCEDE POUR EFFECTUER UNE ANALYSE DE SALIVE

(30) Priorität: 14.07.2000 DE 10034647
(43) Veröffentlichungstag der Anmeldung: 16.04.2003
(73) Patentinhaber: 3M Espe AG, 82229 Seefeld (DE)
(72) Erfinder: WAGNER, Ingo, 82237 Steinebach (DE); HAEBERLEIN, Ingo, 82362 Weilheim (DE); PEUKER, Marc, 86938 Schondorf (DE)
(74) Vertreter: Strehl Schübel-Hopf & Partner
(86) Internationale Anmeldenummer: PCT/EP2001/007778
(87) Internationale Veröffentlichungsnummer: WO 2002/006820

(56) Entgegenhaltungen:
- EP-A- 0 895 943
- WO-A-98/22025
- GB-A- 2 201 241
- US-A- 3 835 834

## Beschreibung

Die Erfindung betrifft ein Analyseverfahren und eine Vorrichtung zur Durchführung eines Analyseverfahrens, insbesondere von Speichel, das sich insbesondere zur Detektion von kariesauslösenden und/oder parodontitisauslösenden Bakterien, die sich beispielsweise in Speichel und/oder Zahnfleischtaschenflüssigkeit befinden können, eignet.

Bei der Untersuchung der Ursachen von Karies und Parodontitis wurden u.a. einzelne Bakterien und Substanzen identifiziert, die als Auslöser dieser Krankheiten in Frage kommen. Sobald sich derartige Keime im Mund befinden, beginnt die Zerstörung des dentalen Hart- und Weichgewebes.

Wünschenswert wäre es, über ein Verfahren und eine Vorrichtung zu verfügen, mit denen sich auf einfache Weise das individuelle Karies- und/oder Parodontitisrisiko bestimmen lassen, um rechtzeitig eine Behandlung einleiten zu können.

Folglich ist es eine Aufgabe der vorliegenden Erfindung, ein für die obige Anwendung geeignetes einfaches Verfahren und eine geeignete Vorrichtung bereitzustellen.

Diese Aufgabe wird durch ein Verfahren und eine Vorrichtung gelöst, wie sie in den Ansprüchen beschrieben sind.

Folgende Vorrichtungen sind aus dem Stand der Technik bekannt.

In EP-A-0895943 ist eine Vorrichtung zum Lagern und Ausbringen einer fließfähigen Substanz beschrieben. Die sich in der Vorrichtung befindliche Bürste dient zum Auftragen einer Substanz auf eine Behandlungsstelle.

In WO-96/03326 ist beispielsweise ein Behältnis zum Einmalgebrauch beschrieben, das Mulden zur Aufbewahrung eines Medikaments und eines Applikators aufweist. Beide Mulden sind durch eine peelbare Deckfolie vor Kontamination geschützt. In einer Ausführungsform wird das Medikament durch Drücken auf die das Medikament enthaltende Mulde in die den Applikator enthaltende Mulde überführt, um den Applikator zu benetzen.

US-A-3,835,834 betrifft einen Transportbehälter für Kulturmedien. Es wird ausgeführt, dass zum Gebrauch ein saugfähiger Applikator nach Öffnen der sterilen Verpackung und Entnahme des Applikators mit dem Kulturmedium benetzt und wieder in den Transportbehälter zurückgeführt wird. Der Transportbehälter wird anschließend verschlossen und auf den saugfähigen, benetzten Applikator eine Nährlösung aufgebracht, die ein Absterben des Kulturmediums beim Transport verhindern soll.

Erfindungsgemäß wurde gefunden, dass sich Abwandlungen dieser Vorrichtungen zur Durchführung eines Analyseverfahrens eignen.

Die erfindungsgemäß einsetzbare Vorrichtung umfasst mindestens eine erste Kammer, die von einer ersten und einer zweiten Folie gebildet wird. In diese Kammer ist eine Komponente eines Indikators zumindest teilweise eingebracht. Die Vorrichtung umfasst femer eine von der Kammer getrennte Tasche, die ihrerseits eine Vorkammer aufweist oder als eine solche fungiert, wobei die Trennung zwischen der Tasche und der ersten Kammer einen selektiv zu öffnenden Durchgangsbereich aufweist.

Erfindungsgemäß umfasst das Verfahren folgende Schritte:
a) Entnehmen eines Applikators aus der Vorrichtung, die einen Applikator enthält, wobei sich der Applikator entweder in einer nach außen hin offenen Tasche oder in einem geschlossenen Behältnis befindet, das nach Öffnen der Vorrichtung und des Behältnisses und Entnehmen des Applikators, eine nach außen hin offene Tasche bildet,
b) in Kontakt bringen des Applikators mit einer Substanz, beispielsweise Speichel, insbesondere Zahnfleischtaschenflüssigkeit,
c) Einführen des Applikators in die Vorrichtung, vorzugsweise in die Tasche aus der der Applikator entnommen wurde oder in eine separate Öffnung, beispielsweise in die nach dem Öffnen der Vorrichtung gebildete Tasche,
d) ggf. in Kontakt bringen des kontaminierten Applikators aus Schritt b) mit einer Nährlösung, die sich in einer zweiten Kammer befindet und die durch Ausübung von Druck aus dieser Kammer über einen selektiv zu öffnenden Durchgangsbereich auf den Applikator aufgebracht werden kann,
e) Aktivieren der Vorrichtung durch Ausübung von Druck auf eine die indikatorsubstanz enthaltende erste Kammer oder durch Einschieben des Applikators in diese Kammer, wobei der Applikator mit der Indikatorsubstanz in Kontakt gebracht wird,
f) gegebenenfalls Entnehmen des Applikators nach einer Zeitspanne, die ausreichend ist, um ein detektierbares Signal zu erzeugen,
g) gegebenenfalls in Kontakt bringen des Applikators mit einem Stoppreagenz, um die Erzeugung eines detektierbaren Signals nach einer Zeitspanne zu beenden, wobei sich das Stoppreagenz vorzugsweise in einer dritten Kammer befindet,
h) gegebenenfalls Vergleich des detektierbaren Signals mit einem Referenzsignal, beispielsweise einer Farbtafel, die vorzugsweise auf der Vorrichtung angebracht ist,
i) gegebenenfalls Durchführung einer Postivkontrolle zur Überprüfung der Funktionsfähigkeit des Testverfahrens, insbesondere falls kein oder nur ein schwer detektierbares Signal vorliegt.

Als Applikator ist jeder manuell handhabbare Gegenstand zu verstehen, der geeignet ist, mit einer anderen Substanz in Kontakt gebracht zu werden.

Der Applikator ist vorzugsweise pinsel- oder tupferartig gestaltet. Ein Applikationsinstrument mit einer kugelförmigen, Bürsten- oder Pinselhaare tragenden Spitze hat sich hierbei als anwendbar erwiesen. Insbesonder Wattestäbchen und Schwämme haben sich als besonders günstig anwendbar erwiesen. Gegebenenfalls weist das Wattestäbchen an beiden Enden saugfähige Bereiche auf. Auf den zweiten saugfähigen Bereich können beispielsweise Substanzen aufgebracht sein, die zur Durchführung einer Positivkontrolle erforderlich sind. Ferner können Pipetten oder Spatel als Applikationsinstrument verwendet werden. Gegebenenfalls weist der Applikator auch eine Spitze auf, die es erlaubt, beispielsweise durch die Haut hindurch Probensubstanz zu entnehmen.

Ferner ist es bei Verwendung des Applikators günstig, wenn die Tasche durch das Applikationsinstrument nach außen abgedichtet wird.

Dies kann beispielsweise dadurch erreicht werden, dass der Applikator am Schaft ein selbstschneidendes Gewinde aufweist. Das Ein- und Ausführen des Applikators in oder aus der Tasche erfolgt dann durch Drehen des Applikators.

Eine derartige Ausführungsform ermöglicht auch ein kontrolliertes Durchmischen der sich in der Tasche bzw. der Vorkammer befindlichen Substanzen durch Drehen des Applikators, ohne dass Substanz durch die Öffnung der Tasche austreten kann.

Bei dieser Ausführungsform ist es günstig, wenn die Tasche zumindest im Öffnungsbereich eine metallhaltige Folie aufweist, in die ein Gewinde eingeschnitten werden kann.

Unter Tasche ist die Gestaltung eines Abschnitts der Vorrichtung in Form eines nach einer Seite hin offenen Behältnisses zu verstehen, das geeignet ist, einen Applikator aufzunehmen.

Der Applikator kann sich von Anfang an in dieser Tasche befinden und wird nach Benetzung mit einer Probensubstanz wieder in diese zurückgeführt.

Denkbar ist aber auch, dass der Applikator in ein verschlossenes Behältnis eingelegt ist, aus dem er nach Öffnen der Vorrichtung, vorzugsweise in steriler Form, entnommen wird.

Vorteilhaft ist in diesem Fall, wenn beim Öffnen der Vorrichtung zur Entnahme des Applikators gleichzeitig eine Tasche mit einer Vorkammer geöffnet wird, in die der Applikator nach Benetzen mit der Probensubstanz eingeführt wird.

Unter den Begriff Indikatorsubstanz fallen alle Substanzen, die geeignet sind, mit einer anderen Substanz ein detektierbares Signal zu erzeugen, vorzugsweise in optisch wahrnehmbarer Form.

Geeignete Indikatorsubstanzen umfassen beispielsweise pH-Indikatoren (beispielsweise Bromphenolblau, Kongorot, Bromkresolgrün, Oregon Green-Derivate, Rhodol-Derivate), Redox-Indikatoren (beispielsweise Methylenblau, 5-Cyano-2,3-ditolyl-tetrazolium-chlorid (CTC), 2-(4-lodophenyl)-3-(4-nitrophenyl)-5-phenyl-2H-tetrazolium-chlorid (INT), 8-Dimethylamino-2,3-benzophenoxazin (Meldola's blue), 1-Methoxy-phenazin-methosulphat (MPMS), 5-(3-Carboxymethoxyphenyl)-2-(4,5-dimethylthiazolyl)-3-(4-sulphophenyl)-tetrazolium (MTS), 3-(4,5-Dimethylthiazol-2-yl)-2,5 diphenyltetrazolium-bromid (MTT), 3,3'-(3,3'-Dimethoxy-4,4'-biphenylen)-bis[2-(4-nitrophenyl-5-phenyl)]-2H-tetrazolium-chlorid (NBT), Nitro-tetrazolium-violet (NTV), Phenazin-methosulphat (PMS), Natrium-3'-[1-[(phenylamino)carbonyl]-3,4-tetrazolium]bis(4-methoxy-6-nitro)-benzolsulfonsäure (XTT), Phenazin-ethosulfat (PES, WST-1), Fluoreszenzindikatoren (beispielsweise Oregon Green 488 BAPTA, Calcium green, Calcium Orange, Calcium Crimson), Chemolumineszenz-Indikatoren, Vitalitätsindikatoren (beispielsweise 5-Bromo-2'deoxyuridin), Farbindikatoren (beispielsweise p-Nitroanilin-Derivate, 2-Naphthylamin-Derivate, 7-Amino-4-methylcoumarin- Derivate, 7-Amino-4-chloromethylcoumarin-Derivate, 6-Aminoquinolin-Derivate, Rhodamin-Derivate, 5,5'-Dithiobis-(2-nitrobenzoesäure), Monobrombiman- Derivate, Tetramethylrhodamin-Derivate, Eosin-Derivate, Erythrosin-Derivate, Texas-Red-Derivate, Coumarin-Derivate, Pyridyloxazol-Derivate, Benzofurazan-Derivate, Naphtalin-Derivate, Didansyl-Cystein, Dansyl- Aziridin, Pyren-Derivate, Coomassie-Blau).

Darüber hinaus können die Indikatorsubstanzen kovalent an Enzyme, Proteine, Glycoproteine, Lipopolysaccharide, Polysaccharide, polyklonale und monoklonale Antikörper, DNA, RNA, Zellorganelle oder Mikroorganismen gebunden sein. Femer können die Indikatorsubstanzen an Silikone, Polyether, Alginate, Polyethylene, Polypropylene, Poly(meth)acrylate, Polyurethane, Polycarbonate, Polysulfide, Polyvinylchloride, Polyvinylpyrrolidone, Polyvinylalkohole oder Kautschuk, Gelatine, Wachse, Fette gebunden sein.

Unter den Begriff Probensubstanz bzw. Markerverbindung fallen alle Substanzen, die geeignet sind, mit der Indikatorsubstanz ein detektierbares Signal zu erzeugen und auf Munderkrankungen und Heilungsprozesse hinweisen, vorzugsweise in optisch wahrnehmbarer Form. Der Begriff Markerverbindung umfasst auch Mikroorganismen.

insbesondere sind mit Markerverbindungen Substanzen mit organischen Bestandteilen gemeint.

Die Markerverbindungen liegen vorzugsweise in flüssiger Form vor. Denkbar sind aber auch die Aggregatszustände fest und gasförmig.

Vorzugsweise befinden sich die Markerverbindungen in Körperflüssigkeiten, die von Drüsen abgesondert werden, wie Speichel, Schweiß, Tränen. Umfasst sind aber auch alle sonstigen Körperflüssigkeiten und Ausscheidungsprodukte, wie Zahnfleischtaschenflüssigkeit, Urin, Blut, Vaginalflüssigkeit und Sperma.

Zu den diagnostizierbaren Munderkrankungen gehören auch Karies, Early Onset Parodontitis, präpubertale Parodontitis, juvenile Parodontitis, schnell verlaufende progressive Parodontitis (RPP), adulte Parodontitis, refraktäre Parodontitis, Gingivitis, Halitosis, Infektionen mit Candida albicans, Candida krusei, Candida glabrata, Candida lusitaniae, Candida dubliniensis, Krebs, aphtöse Stomatitis, Gingivostomatitis.

Im Mundraum können sich Halitose auslösende Bakterien befinden, die flüchtige Schwefelverbindungen, wie Mercaptane oder Schwefelwasserstoff freisetzen. Daneben sind dissimilatorische sulfatreduzierende Bakterien bekannt, deren Schwefelwasserstoffbildung mit der Sulfatreduktion korreliert ist.

Durch die Anwendung des erfindungsgemäßen Verfahrens lassen sich die Bildungsraten von Schwefelwasserstoff und Mercaptanen, bevorzugt Methylmercaptan in Zahnfleischtaschen messen. Darüber hinaus können die bakteriellen Enzymaktivitäten, bevorzugt Methionin-γ-lyase, besonders bevorzugt Cysteindesulfhydrase, die die Bildung der flüchtigen Schwefelverbindungen katalysieren, als Maß für die Halitosisaktivität von Zahnfleischtaschen gemessen werden. Darüber hinaus kann die Gegenwart der für die Freisetzung verantwortlichen Bakterien, bevorzugt Fusobakterien, Porphyromonas, Veillonella, Clostridium und Treponema bestimmt werden.

Die verschiedenen Formen der Parodontitis sind kausal mit der Infektion durch Actinobacillus Actinomycetemcomitans, Bacterioides forsythus, Campylobacter rectus, Capnocytophage ochracea, Capnocytophage gingivalis, Eikenella corrodens, Fusobacterium nucleatum, Porphyromonas asaccharolyticus, Porphyromonas gingivalis, Prevotella dentalis, Prevotella intermedia, Prevoltella nigrescens, Treponema denticola verbunden.

Durch die Anwendung des erfindungsgemäßen Verfahrens lassen sich Gegenwart und Menge der Bakterien bestimmen. Hierfür eignen sich spezifische polyklonale Antikörper und deren Subklassen oder monoklonale Antikörper, die gegen Oberflächenantigene dieser Bakterien, beispielsweise Fimbriae, extrazelluläre Polysaccharide, Adhesine gerichtet sind.

Durch die Anwendung des erfindungsgemäßen Verfahrens können weiterhin Enzymaktivitäten gemessen werden, die einen Hinweis auf die Gegenwart und metabolische Aktivität eines Bakteriums oder einer Gruppe der genannten Parodontitis erzeugenden Bakterien ergeben. Die Trypsin-ähnliche Protease-Aktivität, bevorzugt die Dipeptidylpeptidase-Aktivität, besonders bevorzugt Arg-Gingipain-Aktivität und Lys-Gingipain-Aktivität, wird diagnostisch genutzt. Zur Bestimmung der Arg-Gingipain-Aktivität können synthetische Peptide eingesetzt werden, die mindestens ein Arg-Rest (in P1-Position) neben der detektierbaren Abgangsgruppe enthalten. Zur Bestimmung der Lys-Gingipain-Aktivität können synthetische Peptide eingesetzt werden, die mindestens ein Lys-Rest (in P1-Position) neben der detektierbaren Abgangsgruppe enthalten. Neben p-Nitroanilin-Derivaten, beispielsweise Nα-Benzoyl-DL-arginin-p-nitroanilid oder Leucin-threoninarginin-p-nitroanilid, 2-Naphtylamin-Peptidderviaten, beispielsweise Nα-Benzoyl-DL-arginin-β-naphtylamid können 6-Aminoquinolin-Peptidderivate, Rhodamin-Peptidderivate sowie Cumarin-Peptidderivate, beispielsweise 7-Amido-4-methylcumarin, wie N-t-Boc-Val-Pro-Arg-7-Amido-4-methylcumarin und 7-Amino-4-chloromethylcumarin, wie N-t-Boc-Val-Pro-Arg-7-Amido-4-chloromethylcumarin als detektierbare Abgangsgruppen eingesetzt werden.

Durch die Anwendung des erfindungsgemäßen Verfahrens lassen sich mit polyklonalen Antikörpern und deren Subklassen oder monoklonalen Antikörpern die bakteriellen Substanzen diagnostizieren, die zur Induktion von Zytokinen führen, Bevorzugt werden Antikörper gegen Lipopolysaccharide, Lipoarabinomannan, Peptidoglycane, Teichonsäurederivate, extrazelluläre Polysaccharide und Lipid A.

Durch die Anwendung des erfindungsgemäßen Verfahrens kann auch die durch Parodontitiserreger induzierte Zytokininbildung mit polyklonalen Antikörpern und deren Subklassen oder monoklonalen Antikörpern diagnostiziert werden. Antikörper gegen die Interleukine IL-1, IL-2, IL-3, IL-4, IL-5, IL-6, IL-7, IL-8, Tumomecrosisfaktor TNFα, Interferone α,β,γ, Colony-forming Faktoren M-CSF, Wachstumsfaktoren EGF, TGFα und Chemokine MCP können eingesetzt werden.

Durch die Anwendung des erfindungsgemäßen Verfahrens kann die Zerstörung parodontalen Gewebes über die Enzymaktivitäten von alkalischer Phosphatase, Arylsulfatase, Aspartataminotransferase, β-Glucuronidase, Cathepsine (G,B,D), Elastase, Hyaluronidase, Lactatdehydrogenase, Lysozym, Matrixmetalloproteinasen (Kollagenasen, Gelatinasen), Tissue Inhibitors Metalloproteinases (TIMP), Stromelysin, Lactoferrin, Tryptase und Myeloperoxidase diagnostiziert werden.

Durch die Anwendung des erfindungsgemäßen Verfahrens können mit polyklonalen Antikörpern und deren Subklassen oder monoklonalen Antikörpern die molekularen Marker für Gingivitis diagnostiziert werden. Zu diesen gehören Zytokine, beispielsweise Interleukine IL-1, IL-2, IL-4, IL-6, TNFα und Arachidonsäurederviate, beispielsweise Prostaglandin E₂.

Karies ist kausal mit der Infektion durch Streptococcus salivarius salivarius, Streptococcus vestibularis, Streptococcus thermophilus, Streptococcus mutans, Streptococcus rattus, Streptococcus sobrinus, Streptococcus cricetus, Streptococcus downei, Streptococcus macacae, Streptococcus ferus, Streptococcus milleri,Streptococcus anginosus, Streptococcus constellatus, Streptococcus intermedius, Strepto-coccus mitis, Streptococcus oralis, Streptococcus sanguis, Streptococcus gordonii, Strepto-coccus parasanguis, Streptococcus crista, Streptococcus mitior, Lactobacillus acidophilus, Lactobacillus alimentarius, Lactobacillus brevis, Lactobacillus buchneri, Lactobacillus casei, Lactobacillus paracasei ss paracasei, Lactobacillus paracasei ss rhamnosus, Lactobacillus paracasei ss tolerans, Lactobacillus delbrueckii, Lactobacillus delbrueckii ss lactis, , Lacto-bacillus delbrueckii ss delbrueckii, Lactobacillus delbrueckii ss bulgaricus, Lactobacillus endocarditis, Lactobacillus fermentum, Lactobacillus gasseri, Lactobacillus pseudoplantarum, Lactobacillus rhamnosus, Lactobacillus salivarius, Actinomyces israelii, Actinomyces odontolyticus, Actinomyces actinomycetemcomitans, Eikenella, Branhamella catarrhalis, Veillonella alcalescens, Veillonella parvula, Actinomyces naeslundii, Rothia dentocariosa verbunden.

Durch die Anwendung des erfindungsgemäßen Verfahrens können mit polyklonalen Antikörpern und deren Subklassen oder monoklonalen Antikörpern, die gegen die verschiedenen Oberflächenantigene dieser Bakterien, beispielsweise Proteine, Lipopolysaccharide, Glycoproteine, Fimbriae, extrazelluläre Pollysaccharide, Adhesine, Lipoteichonsäurederivate, Glucan-Bindungsproteine, Collagen-Bindungsproteine gerichtet sind, Gegenwart und Menge der kariogenen Bakterien diagnostiziert werden.

Durch die Anwendung des erfindungsgemäßen Verfahrens können extrazelluläre Enzymaktivitäten kariogener Bakterien diagnostiziert werden, beispielsweise Proteasen, bevorzugt Glucosyltransferasen, Glucanase, Fructosyltransferase, Fructanase.

Durch die Anwendung des erfindungsgemäßen Verfahrens können Stoffwechselprodukte kariogener Bakterien diagnostiziert werden, beispielsweise Buttersäure, Ameisensäure, bevorzugt Essigsäure, Propionsäure, Milchsäure, besonders bevorzugt Milchsäure. Die mit der Säurefreisetzung einhergehende Versauerung des umgebenden Milieus kann darüber hinaus mit pH-Indikatoren, beispielsweise mit Bromphenolblau, Kongorot, Bromkresolblau, bevorzugt Rhodolderivate, besonders bevorzugt Oregon Green-Derivate, nachgewiesen werden.

Als Folge der Versauerung des pHs im umgebenden Milieu, wie Plaque, werden aus der Zahnhartsubstanz Calciumionen herausgelöst. Durch die Verwendung der erfindungsgemäßen Trägermaterialien und die Anwendung des erfindungsgemäßen Verfahrens kann dieser Prozess mit Calciumindikatoren, beispielsweise Calcium Crimson, bevorzugt Calcium Green, Calcium Orange, besonders bevorzugt Calcium Oregon Green 488 BAPTA, diagnostiziert werden.

Durch die Anwendung des erfindungsgemäßen Verfahrens kann die Zunahme oder die Abnahme der oben genannten Markerverbindungen als Maß für den Erkrankungsverlauf und als Maß für den Heilungsprozess herangezogen werden.

Unter in Kontakt bringen ist die Aufnahme einer Menge an Substanz mit dem Applikator zu verstehen, die ausreichend ist, mit der Indikatorsubstanz ein detektierbares Signal zu erzeugen.

Die Aufnahme kann durch physikalische und/oder chemische Vorgänge, wie Adsorption, Absorption, Adhäsion, Aufsaugen, Benetzen und/oder Diffusion erfolgen.

Die Erfindung weist dabei folgende Vorteile auf:

Das erfindungsgemäße Verfahren erlaubt eine schnelle, unkomplizierte Analyse von Substanzproben.

Dadurch, dass sich alle für die gewünschte Analyse benötigten Hilfsmittel in einer Vorrichtung befinden, ist eine Verwechslung von für die Analyse benötigten Substanzen ausgeschlossen.

Ferner ist durch die Vorrichtung sichergestellt, dass die Indikatorsubstanz vor ihrer Verwendung zur Analyse nicht kontaminiert wird.

Das erfindungsgemäße Verfahren in Verbindung mit der zugehörigen Vorrichtung ermöglicht die Durchführung einer Analyse von Substanzen ohne zeitaufwendiges Aufschrauben und Ausbringen von Indikatorlösungen auf entnommene Proben und kann auch von technisch und fachlich nicht vorgebildeten Personen durchgeführt werden.

Ein weiterer Vorteil besteht darin, dass alle für die gewünschte Analyse benötigten Hilfsmittel in den benötigten Mengenverhältnissen in der Vorrichtung vorliegen. Somit ist eine fehlerhafte Rezeptur von für die Analyse benötigten Substanzen ausgeschlossen.

Die bei dem erfindungsgemäßen Verfahren eingesetzte Vorrichtung ist vorzugsweise zum Einmalgebrauch bestimmt.

Vorteilhaft kann sein, wenn sich auf der Vorrichtung ein Bereich befindet, der einen Vergleich des erzeugten Signals mit einem Referenzsignal ermöglicht.

Ein solches Referenzsignal kann beispielsweise in Form einer farblichen Skala aufgedruckt sein, die auf das von der Indikatorsubstanz erzeugte Signal abgestimmt ist.

Vorzugsweise weist die Vorrichtung in dem Bereich, in dem die Analysenreaktion stattfindet, ein durchsichtiges Fenster auf, das die Beobachtung der Analysenreaktion ermöglicht.

Gegebenenfalls wird dieses Fenster erst nach Abziehen einer Schutzfolie sichtbar, vorzugsweise beim Öffnen der Vorrichtung.

Die erfindungsgemäße Vorrichtung weist gegebenenfalls weitere Kammern auf, vorzugsweise eine zweite und eine dritte Kammer, die ebenfalls jeweils über einen selektiv zu öffnenden Bereich mit der Tasche oder einer anderen Kammer verbunden sind. In diese weiteren Kammern können für das Verfahren neben der eigentlichen Indikatorsubstanz gegebenenfalls erforderliche zusätzliche Substanzen eingebracht werden.

Diese zusätzlichen Substanzen können je nach Bedarf durch Ausübung von Druck auf die jeweilige Kammer durch den selektiv zu öffnenden Bereich in eine andere Kammer oder in die Tasche überführt werden.

Eine derartige Ausführungsform ermöglicht die Verwendung der Vorrichtung für eine Vielzahl von Indikatorsubstanzen, die zwar allein für sich stabil und lagerfähig, für den Einsatz in der Analyse aber zusätzlich einer Aktivierung durch Zugabe anderer Substanzen bedürfen.

Solche für die eigentliche Indikatorsubstanz gegebenenfalls zusätzlich erforderliche Substanzen umfassen Puffersubstanzen, wie Phosphate, beispielsweise Natriumphosphat, Natriumhydrogenphosphat, Natriumdihydrogenphophat, Kaliumphosphat, Kaliumhydrogenphosphat, Kaliumdihydrogenphosphat, Pyrophosphat, Carbonate, beispielsweise Natriumcarbonat, Kaliumcarbonat, Natriumhydrogencarbonat, Kaliumhydrogencarbonat, Essigsäure/Acetat, Citronensäure/Citrat, Diethylbarbitursäure, Tris(hydroxymethyl)aminomethan (TRIS), Glycin, Glycylglycin, N-(2-Acetamido)-2-aminoethansulfonsäure (ACES), N-(2-Acetamido)iminodiacetat(ADA), N,N-Bis(2-hydroxyethyl)-2-aminoethansulfonsäure (BES), N,N-Bis(2-hydroxyethyl)glycin (BICINE), 2,2-Bis-(hydroxyethyl)-iminotris(hydroxymethyl)methan (BIS-TRIS), 2-(Cyclohexylamino)ethansulfonsäure (CHES), 2-[4-(2-Hydroxyethyl-1-piperazin)]ethansulfonsäure (HEPES), 3-[4-(2-Hydroxyethyl-1-piperazinyl)]propansulfonsäure (HEPPS), 2-Morpholinoethansulfonsäure (MES), 3-Morpholinopropansulfonsäure (MOPS), Piperazin-1,4-bis(2-ethansulfonsäure) (PIPES), N-[Tris(hydroxymethyl)-methyl]-2-aminoethansulfonsäure (TES), N-[Tris(hydroxymethyl)-methyl]-glycin (TRICINE), Säuren, wie Schwefelsäure, schweflige Säure, Phosphorsäure, Salzsäure, Essigsäure, Salpetersäure, Basen, wie Natriumhydroxid, Kaliumhydroxid, Lithiumhydroxid, Ammoniak, Calciumhydroxid, Magnesiumoxid, Lösungsmittel, wie Wasser, Methanol, Ethanol, Isopropanol, Propanol, Glycerin, Dimethylsulfoxid, Tetrahydrofuran, Aceton, Butanon, Cyclohexan, Toluol, Methylenchlorid, Chloroform, Alkane, Essigsäureethylester, Salze, wie Magnesiumchlorid, Magnesiumsulfat, Magnesiumnitrat, Calciumchlorid, Calciumsulfat, Calciumnitrat, Eisen(III)chlorid, Eisen(II)chlorid, Ammoniumsulfat, Natriumchlorid, Kaliumchlorid, Natriumphosphate, Kaliumphosphate, Coenzyme, Vitamine, Substrate für Enzyme, wie Zuckerphosphate, Milchsäure, Pyruvat, Essigsäure, Acetat, Propionsäure Propionat, Lipopolysaccharide, Toxine, Peptide, Antikörper, wie polyklonale und monoklonale Antikörper und deren Subklassen und/oder Enzyme, die sich in folgende Klassen unterteilen lassen, wobei die folgende Aufzählung beispielsweise und nicht limitierend für die Erfindung ist:
- Oxidoreductasen und deren Unterklassen, beispielsweise Dehydrogenasen, wie Lactatdehydrogenase, Oxidasen, Peroxidasen, Reductasen, Monooxygenasen, Dioxygenasen;
- Transferasen und deren Unterklassen, beispielsweise C₁-Transferasen, Glycosyl-Transferasen, wie Glycosyltransferasen, Fructosyltransferasen, Aminotransferasen, Phospho-Transferasen;
- Hydrolasen und deren Unterklassen, beispielsweise Esterasen, Glycosidasen, wie Glucanase, Fructanase, Peptidasen, beispielsweise Dipeptidylpeptidasen Arg-Gingipain, Lys-Gingipain, Collagenasen, Gelatinasen, Cathepsine, Elastase, Amidasen;
- Lyasen und deren Unterklassen, beispielsweise C-C-Lyasen, C-O-Lyasen, C-N-Lyasen, C-S-Lyasen;
- Isomerasen und deren Unterlassen, beispielsweise Epimerasen, cis-translosmerasen, intramolekulare Transferasen;
- Ligasen und deren Unterklassen, beispielsweise C-C-Ligasen, C-O-Ligasen, C-N-Ligasen, C-S-Ligasen.

Man kennt heute über 2000 verschiedene Enzyme. Zu ihrer Klassifizierung wurde ein System entwickelt, das Wirkungs- und Substratspezifität berücksichtigt. Daraus ergibt sich, dass zu jedem Enzym spezifische Substrate und/oder Coenzyme (beispielsweise NAD(P), NAD(P)H, FAD, FMN, Liponamid, Ubichinon, Häm, ATP, ADP, AMP, GTP, GDP, GMP, UTP, UDP, UMP, CTP, CDP, CMP, Coenzym A, Thiamindiphosphat, Pyridoxalphosphat, Biotin, und/oder Tetrahydrofolat) gehören.

Diese spezifischen Substrate und/oder Coenzyme sind üblicherweise vorhanden, wenn beispielsweise ein oder mehrere Enzyme als Markersubstanz dienen. Umgekehrt gilt natürlich, dass spezifische Enzyme als erforderliche Substanzen verwendet werden können, wenn spezifische Substrate, beispielsweise Zuckerphosphate, Milchsäure/Lactat, Pyruvat, Essigsäure/ Acetat, Propionsäure/Propionat, Ameisensäure/Formiat, Peptide, synthetische Peptide als Probensubstanzen bzw. Markersubstanzen dienen.

Die sich in der Vorrichtung befindlichen, für die Analyse notwendigen Substanzen können in flüssiger oder pulvriger Form vorliegen. Vorzugsweise liegen sie in einer Form vor, die das in Kontakt bringen der Indikatorsubstanzen und/oder Stoppreagenzien mit der Probensubstanz, die sich auf dem Applikator befindet durch den oder die selektiv zu öffnenden Durchgangsbereiche erlaubt. Vorzugsweise liegen die eingesetzten Reagenzien in gelöster Form vor.

Gegebenenfalls ist es nötig, die Probensubstanz auf dem Applikator zu fixieren oder zu stabilisieren. Dies kann durch Umpuffern, pH-Änderung, Veränderung der Ionenstärke, Zugabe von Inhibitoren gegen enzymatischen Abbau, Stabilisatoren und/oder Fixierungsmittel erfolgen.

Die benötigten Substanzen befinden sich entweder in einer weiteren Kammer, die über einen selektiv zu öffnenden Durchgangsbereich mit dem Bereich verbindbar sind, in dem sich der Applikator befindet oder in einem versiegelten Behältnis, das sich in einer Kammer befindet und das beim Aktivieren der Vorrichtung aufplatzt.

Gegebenenfalls weist die Vorrichtung einen Außenschichtbereich und einen davon verschiedenen Innenschichtbereich auf, der die Kammern bildet. Der Außenschichtbereich kann peelbar ausgebildet sein, so dass sich der Außenschichtbereich zumindest teilweise abziehen lässt.

Die Verwendung unterschiedlich beschaffener Folien für den Außenschichtbereich einerseits und die die Kammer bildenden Folien andererseits erlaubt eine vor Ingebrauchnahme der Vorrichtung sichere Lagerung der Substanzen.

Darüber hinaus bleiben die zu mischenden Substanzen im Lagerzustand vor äußeren Einflüssen, wie Lichteinfall oder auch ungewollter Druckbelastung besser geschützt.

Ist eine der die Kammer bildenden Folien durchscheinend, kann nach Abziehen des peelbaren Außenschichtbereichs die ablaufende Reaktion zwischen zu analysierender Substanz und Indikatorsubstanz und/oder das Entleeren der Kammer in die Tasche beispielsweise optisch verfolgt werden.

Je nach in der Vorrichtung enthaltender Indikatorsubstanz kann die Innenschicht der Folie, die mit der Indikatorsubstanz in Kontakt kommt, an die Eigenschaften der Substanz angepasst werden. Dies ermöglicht die Verwendung der Vorrichtung zur Lagerung für eine Vielzahl von in ihrer Reaktivität unterschiedlicher Indikatorsubstanzen, ohne dass das Verfahren zur Herstellung der Vorrichtung insgesamt umgestellt werden muss.

Gegebenenfalls befindet sich in einer Kammer der Vorrichtung zusätzlich ein separates Kompartiment, vorzugsweise in Form eines Kissens, zur Aufnahme einer Substanz, wie der eigentlichen Indikatorsubstanz, einer Aktivierungssubstanz für die Indikatorsubstanz oder einer Substanz, die für die Reaktion zwischen zu analysierender Substanz und eigentlicher Indikatorsubstanz erforderlich ist.

Solche Substanzen sind beispielsweise Puffersubstanzen, Säuren, Basen oder Lösungsmittel, wie sie bereits oberhalb beschrieben wurden.

Das Vorhandensein eines derartigen weiteren Kompartiments verhindert, dass diese Substanz im Lagerzustand unmittelbar weder mit dem selektiv zu öffnenden Durchgangsbereich noch mit der die Kammer bildenden Folien in Kontakt kommt.

Dadurch wird eine Beschädigung der Vorrichtung, insbesondere der die Vorrichtung bildenden Folien erschwert. Dies ermöglicht eine Verlängerung der Lagerzeit der die Substanz enthaltenden Vorrichtung.

Hierbei hat sich die Verwendung von die Diffusion flüchtiger Substanzen stark beeinträchtigenden oder verhindernden Materialien zur Herstellung des Behältnisses als vorteilhaft erwiesen.

Die erste und zweite Folie sind gegebenenfalls Mehrschichtfolien. Sie weisen einen gegebenenfalls zumindest teilweise peelbaren Außenschichtbereich und einen Innenschichtbereich auf.

Als Folien werden vorzugsweise solche verwendet, die eine ausreichende Diffusionsdichtigkeit aufweisen.

Je nach Beschaffenheit der zu lagernden Substanz sollten die Folien auch beständig gegen agressive, beispielsweise ätzende und/oder Lösungsmitteleigenschaften aufweisende Stoffe sein.

Die Folienbestandteile können gewählt sein aus Kunststoff-, Metall- und keramischen Folien.

Als Kunststoffe zur Herstellung von Folien sind beispielsweise denkbar: PE, PP, PTFE, PET, PA, PBT, PVC, EVA.

Als Metalle zur Herstellung von Folien sind beispielsweise denkbar: Al, Sn, Au, Ag, Fe, Cu.

Unter keramischen Folien sind Folien zu verstehen, die beispielsweise eine SiOx-haltige Schicht aufweisen.

Der Folienaufbau ist grundsätzlich beliebig und orientiert sich an der Beschaffenheit der zu lagernden Substanzen.

Als vorteilhaft hat sich ein Folienaufbau mit der Abfolge von außen nach innen PET, Al, PET, PE oder PP, Al, PET, PE, gegebenenfalls auch ohne PET-Folie als Mittelfolie, erwiesen.

Zwischen den Folien können sich ferner adhäsive Zusätze befinden.

Als adhäsive Zusätze sind denkbar: Kaschierkleber oder Extrusionskaschiermedien.

Der gegebenenfalls vorhandene peelbare Außenschichtbereich der ersten Folie ist vorzugsweise lichtundurchlässig.

Der innenschichtbereich der ersten Folie ist vorzugsweise lichtdurchlässig und gegebenenfalls flexibler als der Außenschichtbereich.

Als vorteilhaft für den Außenschichtbereich hat sich die Abfolge von außen nach innen PET, Al oder PP, Al erwiesen.

Die Trennung zwischen der oder den Kammern und der Tasche ist bezüglich des Abstandes sowie bezüglich der Festigkeit der Haftung so gestaltet, dass sie eine Sollbruchstelle bilden können.

Der Durchgangsbereich ist so beschaffen, dass er im Lagerzustand einen dichten Verschluss bildet, sowohl zur Tasche hin als auch zur gegebenenfalls vorhandenen zweiten und/oder dritten Kammer.

Eine solche Sollbruchstelle lässt sich beispielsweise durch Heißsiegeln oder Verkleben erreichen, wobei beim Heißsiegeln ein im Vergleich zu den anderen Siegelbereichen unterschiedlicher Energieeintrag, vorzugsweise niedrigerer Energieeintrag erfolgt. Dieser kann über Temperatur, Druck und/oder Haltedauer geregelt werden.

Eine andere Möglichkeit besteht darin, im Bereich der Sollbruchstelle zwischen die erste und die zweite Folie die Haftung herabsetzende Fremdpartikel, wie Peelfolienstanzlinge oder Hotmeltklebepunkte einzubringen. In diesem Fall werden als Ober- und Unterfolie vorzugsweise festversiegelnde Folien eingesetzt.

Die Kammer oder die Kammern sind vorzugsweise rund (kreisrund oder oval) gegebenenfalls aber auch eckig (quadratisch, rechteckig oder dreieckig) ausgebildet.

Die Kammern weisen ihrem Zweck entsprechend bestimmte Volumen auf, so dass bei vorschriftsmäßiger Anwendung und mehrerer zu mischender Komponenten eine homogene Durchmischung erfolgen kann. Die der Tasche benachbarte Kammer hat dabei vorzugsweise ein Volumen, das geeignet ist, die gesamte Menge an zu mischender oder gemischter Substanz aufzunehmen.

Das Kammervolumen kann im Bereich von wenigen Zehntel Millilitern, beispielsweise 0,01 ml bis etwa 50 ml liegen, bevorzugt ist ein Bereich von 0,01 bis 10 ml, besonders bevorzugt ein Bereich von 0,01 bis 5 ml.

Eine Kammer weist beispielsweise einen Durchmesser von 5 bis 20 mm auf, das Ausbringinstrument einen Schaftdurchmesser im Bereich von 2 mm.

Die Tasche ist nach einer Seite hin offen und gegebenenfalls so ausgebildet, dass sie ein Ausbringinstrument auch im Lagerzustand aufnehmen kann. Die Tasche kann bei entsprechend kleinem Durchmesser der Taschenöffnung nach außen, beispielsweise in Form einer Kanüle, auch selbst als Applikationsvorrichtung dienen.

Die Vorrichtung umfasst einen Behälter, der eine erste Folie, beispielsweise eine Deckfolie und eine zweite Folie, beispielsweise eine Tiefziehfolie, wie sie bei herkömmlichen Blisterverpackungen Verwendung findet, umfasst.

Die Folien sind mit Ausnahme der die Kammern bildenden Bereiche vorzugsweise flächig miteinander verbunden.

Die Verbindung der ersten mit der zweiten Folie kann beispielsweise durch Heißsiegeln, Kaltsiegeln, Verkleben und/oder Ultraschallschweißen mit Sonotroden, erfolgen.

Ein Mehrschichtaufbau der ersten und zweiten Folie kann durch Laminieren, Kalandrieren, Kaschieren verschiedener Monofolienlagen, gegebenenfalls auch durch Bedampfen, beispielsweise mit Metallen, erreicht werden.

Um die in die Vorrichtung eingebrachten, applizierbaren Substanzen beispielsweise vor Lichteinfall zu schützen, sind die Folien vorzugsweise derart gestaltet, dass sie in einem die Kammer umgebenden Bereich durch zwei in Abstand voneinander angeordnete Siegelnähte miteinander verbunden sind.

Das oder die Behältnisse lassen sich mit den gleichen Verfahren herstellen, die zur Herstellung der Vorrichtung angewandt werden können. Das Behältnis wird dabei vorzugsweise durch Verschweißen, Verkleben oder Versiegeln im Randbereich von kunststoff- oder metallhaltigen Folien hergestellt, so dass vorzugsweise ein kissenförmiges Gebilde entsteht. Die Verbindung der Folien erfolgt derart, dass sich das Behältnis durch Einwirkung von äußerem Druck öffnen lässt, wobei das Behältnis bei Druckeinwirkung vorzugsweise ungerichtet aufplatzen soll.

Zum Gebrauch der Vorrichtung wird zunächst der Applikator aus der Tasche entnommen und mit der zu analysierenden Substanz in Berührung gebracht, vorzugsweise in die zu analysierende Substanz eingetaucht. Der Vorgang des Benetzens des Applikators dauert üblicherweise nur wenige Sekunden.

Zur Analyse von Speichel kann der Applikator entweder in ein mit Speichel gefülltes Gefäß eingetaucht werden oder im einfachsten Fall der Speichel direkt aus dem Mund entnommen werden, dadurch, dass der Applikator im Mund mit Speichel in Berührung gebracht wird.

Zur Analyse der Zahnfleischtaschenflüssigkeit kann der Applikator beispielsweise in eine Zahnfleischtasche eingeführt werden.

Der benetzte Applikator wird in eine Öffnung der Vorrichtung, vorzugsweise in die Tasche, aus der er entnommen wurde, zurückgesteckt.

Anschließend erfolgt die Aktivierung der Indikatorsubstanz, sofern dies erforderlich ist. Dies kann beispielsweise durch Ausübung von Druck auf eine eine Aktivierungssubstanz enthaltende zweite Kammer erfolgen. Die Ausübung von Druck bewirkt, dass die Aktivierungssubstanz über den selektiv zu öffnenden Durchgangsbereich in die Kammer gelangt, in der sich die eigentliche Indikatorsubstanz befindet.

Alternativ kann die Aktivierung auch durch Ausübung von Druck auf die die Indikatorsubstanz enthaltende Kammer selbst erfolgen, wenn sich die Aktivierungssubstanz in einem gegebenenfalls vorhandenen Behältnis in der Kammer befindet. Dieses üblicherweise kissenförmig ausgebildete Behältnis platzt hierbei vorzugsweise ungerichtet auf.

Die gegebenenfalls aktivierte Indikatorsubstanz wird anschließend mit dem benetzten Applikator in Berührung gebracht. Dies kann dadurch erreicht werden, dass auf die die Indikatorsubstanz enthaltende Kammer von außen Druck ausgeübt wird, was zu einer Öffnung des selektiv zu öffnenden Durchgangsbereichs zu der den Applikator enthaltenden Tasche führt. Die Reaktion zwischen Indikatorsubstanz und Analysensubstanz erfolgt dann in der Tasche.

Alternativ kann der Applikator auch durch den selektiv zu öffnenden Durchgangsbereich in die die Indikatorsubstanz enthaltende Kammer geschoben werden. Die Reaktion findet in diesem Fall in der die Indikatorsubstanz enthaltenden Kammer statt.

Denkbar ist auch, dass der Applikator in eine leere Kammer durch einen ersten selektiv zu öffnenden Durchgangsbereich geschoben wird. Über einen mit dieser Kammer verbundenen weiteren selektiv zu öffnenden Durchgangsbereich wird anschließend die Indikatorsubstanz, gegebenenfalls zusammen mit einem Aktivierungsmittel, in die ursprünglich leere Kammer überführt. Die Reaktion findet in diesem Fall in der ursprünglich leeren Kammer statt.

Nach Beendigung der Reaktion, die üblicherweise in einem Zeitraum von wenigen Sekunden bis zu wenigen Minuten abläuft, wird der Applikator gegebenenfalls aus der Vorrichtung entnommen. Je nach verwendeter Indikatorsubstanz erhält man ein detektierbares Signal, vorzugsweise einen optisch registrierbaren Farbumschlag.

Zum Beenden der Bildung des detektierbaren Signals kann aus einem weiteren Kissen ein Stopp-Reagenz eingebracht werden.

Geeignete Stop-Reagenzien sind beispielsweise Säuren, wie Schwefelsäure, schweflige Säure, Phosphorsäure, Salzsäure, Essigsäure, Salpetersäure, Citronensäure, Ascorbinsäure, Basen, wie Natriumhydroxid, Kaliumhydroxid, Lithiumhydroxid, Ammonik, Calciumhydroxid, Inhibitoren, wie Jodessigsäure, Monbrombimanderivate, Dansyl-Derivate, Wasserstoffperoxid. Wenn zu den erforderlichen Substanzen ein oder mehrere Enyzme gehören, können prinzipiell die für das jeweilige Enzym bekannten spezifische Inhibitoren verwendet werden; beispielsweise sind Pyruvat oder Phosphatanionen oder Pyrophosphatanionen Inhibitoren der Lactatdehydrogenase.

Ein Vergleich mit einem Referenzsignal, beispielsweise einer Farbskala, die vorzugsweise auf der Vorrichtung angebracht ist, erlaubt die Auswertung der durchgeführten Analyse.

In einer weiteren bevorzugten Ausführungsform umfasst die Vorrichtung Mittel zur Durchführung einer Positivkontrolle für das Testverfahren. Eine solche Positivkontrolle ist beispielsweise dann sinnvoll, wenn der Applikator bzw. der sich auf diesem befindliche Indikator nach Durchführung des Analysenverfahrens nicht oder nur sehr schwach verfärbt. In einem solchen Fall besteht nämlich die Möglichkeit, dass die nachzuweisende Substanz in der untersuchten Probe nicht vorhanden war, oder die Möglichkeit, dass der Test als solcher nicht funktionsfähig war.

Zur Überprüfung, weiche der Möglichkeiten vorliegt, bietet es sich an, den Applikator nach Durchführung des Verfahrens einer Positivkontrolle zu unterziehen.

Eine geeignete Positivkontrolle die bei einem Verfahren zur Detektion von kariesauslösenden und/oder parodontitisauslösenden Bakterien angewandt werden kann, umfasst beispielsweise eine 1 bis 10 mM Lactatlösung, ggf. in einem Puffer aus Glycylglycin (ca. 50 mM) und Natriumhydrogenphosphat (ca. 10 mM), bei einem pH von ca. 9.

Zur Durchführung der Positivkontrolle in dieser Ausführungsform wird der Applikator nach Durchführung des eigentlichen Verfahrens mit Lactat in Kontakt gebracht. Dabei geht Lactat in Lösung und es sollte bzw. muss zum Farbumschlag des Indikators kommen, wenn der Test funktionsfähig war.

Die Substanzen, die für die Positivkontrolle benötigt werden, befinden sich zweckmäßigerweise in einem separaten Behältnis. Das Behältnis kann beispielsweise ein Vlies enthalten, und beispielsweise auf der Rückseite der Vorrichtung angebracht sein. Denkbar ist aber auch das Anbringen des Behältnisses an der Vorrichtung in Form eines abreißbaren Blisters oder die separate Beigabe, beispielsweise in Form eines Teststreifens.

Bevorzugte Ausführungsbeispiele für die Gestaltung der Vorrichtung werden nachstehend anhand der Zeichnungen erläutert.
Figur 1 zeigt eine Ausführungsform der Vorrichtung mit drei parallel angeordneten Kammern.
Figur 2 zeigt eine Ausführungsform der Vorrichtung mit drei Kammern, von denen zwei seriell angeordnet sind.
Figur 3 zeigt eine Ausführungsform der Vorrichtung mit zwei parallel angeordneten Kammern.
Figur 4 zeigt eine Ausführungsform der Vorrichtung, die eine sterile Lagerung des Applikators ermöglicht.

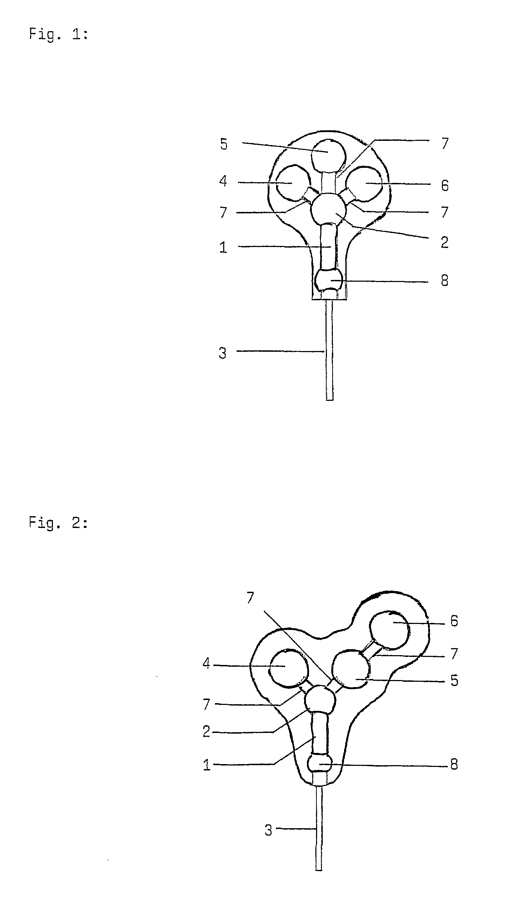

Figur 1 zeigt eine Ausführungsform der erfindungsgemäßen Vorrichtung mit einer Tasche (1), die in eine Vorkammer (2) mündet und in der sich ein Applikator (3) befindet, wobei der Schaft des Applikators aus der Tasche (1) herausragt. Im Öffnungsbereich der Tasche befindet sich gegebenenfalls eine Arretierungshilfe (8) für den Applikator. Die Vorkammer (2) ist gegebenenfalls aus durchscheinendem Material, so dass der Analysenverlauf beobachtet werden kann.

Die Vorrichtung umfasst femer drei Kammern (4, 5, 6), die jeweils über selektiv zu öffnende Durchgangsbereiche (7) in die Vorkammer (2) der Tasche (1) münden. Diese Anordnung wird als parallele Anordnung der Kammern bezeichnet.

Diese Anordnung ist beispielsweise dann günstig, wenn der mit Probensubstanz benetzte Applikator vor dem in Kontakt bringen mit einer Indikatorlösung, die sich in Kammer (4) befindet, zusätzlich mit einer Nährlösung, beispielsweise einer Zuckerlösung, benetzt werden soll, die sich in Kammer (5) befindet. In Kammer (6) befindet sich in diesem Fall ein Stopp-Reagenz.

Eine gegebenenfalls vorhandene Farbskala kann beispielsweise auf der Rückseite der Vorrichtung angebracht sein.

In Figur 2 sind zwei der drei vorhandenen Kammern seriell, d.h. in Reihe angeordnet. Diese Anordnung ist beispielsweise dann vorteilhaft, wenn die Indikatorsubstanz, die sich in Kammer (5) befindet, zunächst aktiviert werden muss. Die Aktivierungslösung befindet sich in diesem Fall vorzugsweise in Kammer (6). Kammer (4) kann entweder eine Nährlösung oder ein Stoppreagenz enthalten.

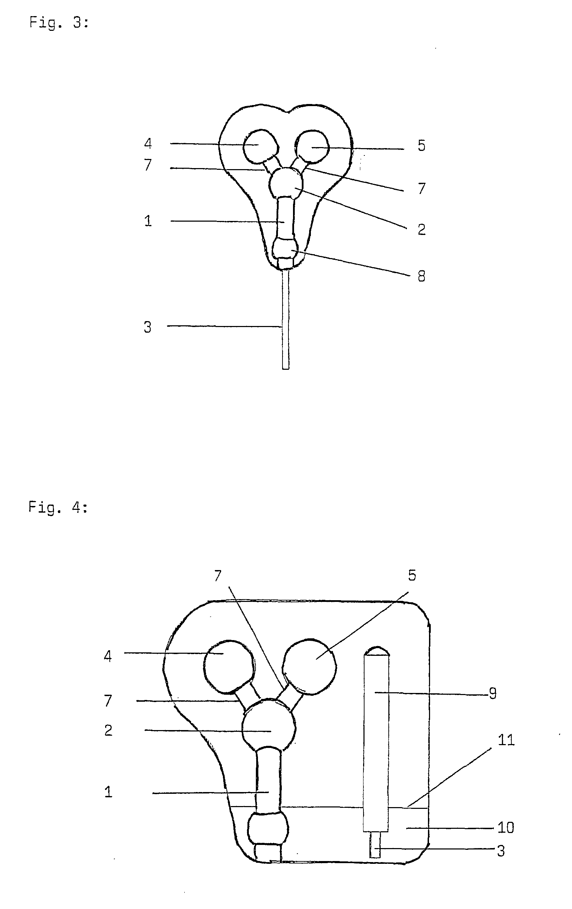

Die Vorrichtung in Figur 3 umfasst nur zwei parallel angeordnete Kammern (4, 5). Denkbar ist auch, dass die Vorrichtung nur über eine Kammer verfügt, in der sich die Indikatoriösung befindet. In diese Kammer kann gegebenenfalls zusätzlich ein weiteres Kompartiment eingelegt sein, das beispielsweise eine Aktivierungslösung für die indikatorsubstanz enthält und beim Aktivieren durch Druckausübung auf die Kammer aufplatzt.

Soll sichergestellt werden, dass der Applikator steril aufbewahrt werden muss, hat sich die Vorrichtung gemäß Figur 4 als vorteilhaft erwiesen. In Figur 4 befindet sich der Applikator (3) in einem Behältnis (9). Dieses Behältnis sowie die Öffnung der Tasche (1) mit der Vorkammer (2) sind im Ausgangszustand durch die Folie (10), die sich vorzugsweise bis zum Falz (11) abziehen lässt, dicht verschlossen. Beim Öffnen der Vorrichtung werden damit sowohl der Schaft des Applikators (3) als auch die Öffnung der Tasche (1) freigelegt.

Das erfindungsgemäße Verfahren eignet sich zur Analyse sämtlicher Substanzen, die den in der Vorrichtung enthaltenen Applikator benetzen können und die mit der in der Vorrichtung enthaltenen Indikatorsubstanz reagieren können.

Vorzugsweise eignet sich das Verfahren zur schnellen und einfachen Analyse von Körperflüssigkeiten, insbesondere von Speichel und Blut und den darin enthaltenden organischen bzw. biochemischen Substanzen.

In einer besonderen Ausführungsform ist das Verfahren zur Analyse von Speichel auf solche darin enthaltende Substanzen geeignet, die zur Ermittlung des individuellen Karies- und/oder Paradontitisrisikos herangezogen werden können.

In einer weiteren besonderen Ausführungsform ist das Verfahren zur Analyse von Speichel auf solche darin enthaltende Substanzen geeignet, die zur Ermittlung des individuellen Parodontitis-Risikos herangezogen werden können.

Für einen solchen Speicheltest haben sich folgende Indikatorsubstanzen als vorteilhaft erwiesen: Farbstoffe die durch proteolytische Aktivität von Proteasesubstraten abgelöst werden. Insbesondere geeignet sind Redox-Farbstoffe, beispielsweise PMS, MTT, PES, CTT, MTS. Geeignet sind aber auch alle vorstehend genannten Indikatorsubstanzen. Umfasst sind auch Peptidderivate des p-Nitroanilins, des 2-Naphthylamins, des 7-Amino-4-methylcoumarins, des 7-Amino-4-chloromethylcoumarins, des 6-Aminoquinolins, des Rhodamins, N-Benzoyl-DL-arginine-2-naphtylamid und Benzoyl-DL-arginin-p-nitroanilid.

Ebenfalls Gegenstand der vorliegenden Erfindung ist ein Kit zur ortspezifischen Bestimmung des individuellen Karies- und/oder Parodontitisrisikos mittels einer Abformmasse, enthaltend diagnostisch wirksame Zusätze.

Bei dem diesen Kit zugrunde liegenden Verfahren wird zunächst das individuelle Kariesrisiko mittels dem vorher beschriebenen Verfahren aus Speichel ermittelt und bei positivem Ergebnis ein Abdruck vom Hartgewebe, das von Speichel umspült wird, vorzugsweise mindestens einer Kieferregion, beispielsweise des Unter- und/oder Oberkiefers, mit einer diagnostischen Abformmasse genommen.
Als diagnostische Abformmassen sind beispielsweise die Massen geeignet, die in der Anmeldung DE-199 26 728 beschrieben werden, insbesondere auf Alginat-, Polyether-, Silikon- oder Polyethersilikonbasis.

Es handelt sich hierbei um härtbare oder filmbildende Trägermaterialien, die diagnostisch nutzbare Zusatzstoffe für die orts- und stoffspezifische intraorale Diagnose in einer bevorzugten Menge von 0,0001 bis 10 Gew.-% enthalten. Als diagnostische Zusätze sind gemäß dieser Anmeldung Farbstoffindikatoren, Antikörper, Enzyme und alle anderen Substanzen geeignet, die dem mit der Entwicklung von diagnostischen Testsystemen vertrauten Fachmann geläufig sind.

Derartige Abformmassen zeigen im ausgehärteten Abdruckmaterial an denjenigen Stellen ein wahrnehmbares Signal, beispielsweise eine Färbung, an denen ein erhöhtes Kariesrisiko vorliegt. Hierzu werden selektiv Stoffwechselprodukte der kariesfördernden Mikroorganismen als aktive Spezies im angewandten Nachweisverfahren benutzt.

Nachfolgend wird die Erfindung an Hand von Beispielen erläutert.

### Beispiel 1

### Speicheltest 1

Verwendet wird eine Vorrichtung mit einer Tasche für den Applikator und einer Kammer. In der Kammer befinden sich 200 µl Diagnoselösung, enthaltend eine Aminoverbindung, ein Coenzym, eine Dehydrogenase und einen Redoxindikator.

Der Applikator wird aus der Vorrichtung entnommen, im Mund des Patienten mit Speichel benetzt und wieder in die Tasche zurückgeführt. Durch Drücken wird die Kammer geöffnet und die Diagnoseflüssigkeit durch den Durchgangsbereich in die Tasche überführt. Durch Drehen des Applikators wird eine vollständige Benetzung der Applikatorspitze mit Diagnoseflüssigkeit gewährleistet. Nach etwa 3 min kann der Applikator entnommen werden und an der Färbung der Applikatorspitze die vorhandene Menge der nachzuweisenden Substanz im Speichel des Patienten evaluiert werden.

### Beispiel 2

### Speicheltest 2

Verwendet wird eine Vorrichtung mit einer Tasche für den Applikator und zwei parallel angeordneten Kammern entsprechend Abb. 3. In der ersten Kammer befinden sich 200 µl Diagnoselösung, enthaltend eine Aminoverbindung, ein Coenzym, eine Dehydrogenase, und einen Redoxindikator. In der zweiten Kammer befinden sich 50 µl Stoppreagenz.

Der Applikator wird aus der Vorrichtung entnommen, im Mund des Patienten mit Speichel benetzt und wieder in die Tasche zurückgeführt. Durch Drücken wird die Kammer mit der Diagnoseflüssigkeit geöffnet und durch den Durchgangsbereich in die Tasche überführt. Durch Drehen des Applikators wird eine vollständige Benetzung der Applikatorspitze mit Diagnoseflüssigkeit gewährleistet. Nach etwa 3 min wird durch Drücken die zweite Kammer mit dem Stoppreagenz geöffnet und das Stoppreagenz durch den Durchgangsbereich in die Tasche überführt. Durch Drehen des Applikators wird eine vollständige Benetzung der Applikatorspitze mit Stoppreagenz gewährleistet.

Der Applikator kann entnommen werden und an der Blaufärbung der Applikatorspitze kann die vorhandene Menge der nachzuweisenden Substanz im Speichel des Patienten evaluiert werden.

### Beispiel 3

### Speicheltest 3

Verwendet wird eine Vorrichtung mit einer Tasche für den Applikator und zwei seriell und einer parallel angeordneten Kammer entsprechend Abb. 2.

In der oberen seriell angeordneten Kammer befinden sich 200 µl Diagnoselösung, enthaltend eine Aminoverbindung. In der unteren seriell angeordneten Kammer befindet sich das Diagnosepulver, enthaltend ein Coenzym, eine Dehydrogenase, einen Redoxindikator und eine zuckerhaltige Verbindung. In der parallel angeordneten Kammer befinden sich 50 µl Säure als Stoppreagenz.

Der Applikator wird aus der Vorrichtung entnommen, im Mund des Patienten mit Speichel benetzt und wieder in die Tasche zurückgeführt. Durch Drücken wird die obere seriell angeordnete Kammer mit der Pufferlösung geöffnet und durch den Durchgangsbereich in die untere seriell angeordnete Kammer mit dem Diagnosepulver überführt.

Durch wechselseitiges Drücken auf die beiden seriell angeordneten Kammern wird die Flüssigkeit zwischen den beiden Kammern hin und herbewegt, wodurch sichergestellt wird, dass das Diagnosepulver vollständig in Lösung geht. Anschließend wird die Diagnoselösung durch den Durchgangsbereich in die Tasche überführt. Durch Drehen des Applikators wird eine vollständige Benetzung der Applikatorspitze mit Diagnoseflüssigkeit gewährleistet. Nach etwa 3 min wird durch Drücken die parallel angeordnete Kammer mit dem Stoppreagenz geöffnet und das Stoppreagenz durch den Durchgangsbereich in die Tasche des Applikators überführt. Durch Drehen des Applikators wird eine vollständige Benetzung der Applikatorspitze mit Stoppreagenz gewährleistet. Der Applikator kann entnommen werden und an der Färbung der Applikatorspitze kann die vorhandene Menge der nachzuweisenden Substanz im Speichel des Patienten evaluiert werden.

### Beispiel 4

### Speicheltest 4

Verwendet wird eine Vorrichtung mit einer Tasche für den Applikator und zwei parallel angeordneten Kammern entsprechend Abb. 3. In der ersten Kammer befinden sich 200 µl Diagnoselösung, enthaltend eine Aminoverbindung, eine Mischung verschiedener Salze, sowie eine peptidhaltige Verbindung. In der zweiten Kammer befinden sich 50 µl Säure als Stoppreagenz.

Der Applikator wird aus der Vorrichtung entnommen, im Mund des Patienten mit Speichel benetzt und wieder in die Tasche zurückgeführt. Durch Drücken wird die Kammer mit der Diagnoseflüssigkeit geöffnet und durch den Durchgangsbereich in die Tasche überführt. Durch Drehen des Applikators wird eine vollständige Benetzung der Applikatorspitze mit Diagnoseflüssigkeit gewährleistet. Durch das Sichtfenster in der Applikatortasche kann die Bildung einer Gelbfärbung beobachtet werden.

Nach etwa 3 min wird durch Drücken die zweite Kammer mit dem Stoppreagenz geöffnet und das Stoppreagenz durch den Durchgangsbereich in die Tasche überführt. Durch Drehen des Applikators wird eine vollständige Benetzung der Applikatorspitze und Durchmischung der Lösung mit Stoppreagenz gewährleistet. Die Reaktion kommt zum Erliegen und die Färbung kann mit einer Farbtafel evaluiert werden.

### Beispiel 5

### Speicheltest 5

Verwendet wird eine Vorrichtung mit einer Tasche für den Applikator und einer Kammer. In der Kammer befindet sich 200 µl Diagnoselösung, enthaltend eine Aminoverbindung, eine Mischung verschiedener Salze und eine peptidhaltige Verbindung.

Der Applikator wird aus der Vorrichtung entnommen, im Mund des Patienten mit Speichel benetzt und wieder in die Tasche zurückgeführt. Durch Drücken wird die Kammer geöffnet und die Diagnoseflüssigkeit durch den Durchgangsbereich in die Tasche überführt. Durch Drehen des Applikators wird eine vollständige Benetzung der Applikatorspitze mit Diagnoseflüssigkeit gewährleistet.

Nach etwa 3 min kann der Applikator entnommen werden und an der Färbung der Applikatorspitze die vorhandene Menge der zu analysierenden Substanz im Speichel des Patienten evaluiert werden.

### Bezugszeichenliste

- 1.: Tasche
- 2.: Vorkammer
- 3.: Applikator
- 4.: erste Kammer
- 5.: zweite Kammer
- 6.: dritte Kammer
- 7.: selektiv zu öffnender Durchgangsbereich
- 8.: Arretierungshilfe
- 9.: Behältnis zur Aufnahme des Applikators
- 10.: Aufziehfolie
- 11.: Öffnungs-Falz

## Patentansprüche

1. Analysenverfahren, umfassend folgende Schritte:
a) Entnehmen eines Applikators aus einer Vorrichtung, umfassend eine Tasche zur Aufnahme eines Applikators und mindestens eine erste Kammer enthaltend eine Indikatorsubstanz, wobei die Kammer mit der Tasche über einen selektiv zu öffnenden Durchgangsbereich verbindbar ist,
b) in Kontakt bringen des Applikators mit einer Probensubstanz,
c) Einführen des Applikators in die Vorrichtung, vorzugsweise in die Tasche,
d) Aktivieren der Vorrichtung durch Ausübung von Druck auf mindestens eine Kammer oder durch Einschieben des Applikators in eine Kammer, wobei der Applikator mit der Indikatorsubstanz in Kontakt gebracht wird,
e) gegebenenfalls Entnehmen des Applikators nach einer Zeitspanne, die ausreichend ist, um ein detektierbares Signal zu erzeugen,
f) gegebenenfalls in Kontakt bringen des Applikators mit einem Stoppreagenz, um die Erzeugung des detektierbaren Signals nach einer Zeitspanne zu beenden, wobei sich das Stoppreagenz in einer weiteren Kammer befindet,
g) Vergleich des detektierbaren Signals mit einem Referenzsignal.
h) gegebenenfalls Durchführung einer Postivkontrolle zur Überprüfung der Funktionsfähigkeit des Analysenverfahrens,
wobei sich der Applikator von Anfang an in der Tasche befinden kann oder in ein verschlossenes Behältnis eingelegt ist.

2. Verfahren nach Anspruch 1, wobei der Applikator nach Schritt c) zunächst in einem Schritt c') mit einer Nährlösung in Kontakt gebracht wird.

3. Verfahren nach.einem der vorstehenden Ansprüche, wobei vor Aktivieren der Vorrichtung gemäß Schritt d) die Indikatorsubstanz durch Vermischen der Indikatorsubstanz mit einer Aktivierungssubstanz aktiviert wird.

4. Verfahren nach einem der vorstehenden Ansprüche, wobei vor Schritt a) die Vorrichtung durch zumindest partielles Ab- oder Aufziehen einer Folie oder eines Folienbestandteiles geöffnet wird.

5. Verfahren nach einem der vorstehenden Ansprüche, wobei es sich bei der Probensubstanz um Speichel handelt und im Anschluss an Schritt g) unter Verwendung einer Abformmasse, enthaltend diagnostisch wirksame Zusätze, ein Abdruck vom Gewebe angefertigt wird, das vom Speichel umspült wird.

6. Verwendung einer Vorrichtung, umfassend eine Tasche zur Aufnahme eines Applikators, einen Applikator, mindestens eine erste Kammer zur Aufnahme einer Indikatorsubstanz, und eine weitere Kammer zur Aufnahme von Puffersubstanzen, Säuren, Basen, Lösungsmitteln, Salzen, Enzymen, Coenzymen, Vitaminen und/oder Stopp-Reagenzien, wobei die Kammer mit der Tasche über einen selektiv zu öffnenden Durchgangsbereich verbindbar ist, zur Durchführung des Verfahrens nach einem der Ansprüche 1 bis 5.

7. Verwendung einer Vorrichtung nach Anspruch 6 zur Durchführung einer Analyse von Speichel zur Detektion von kariesauslösenden Bakterien, und/oder zur Analyse von Zahnfleischtaschenflüssigkeit, insbesondere zur Detektion von Parodontitis auslösenden Bakterien.

8. Vorrichtung, umfassend eine Tasche zur Aufnahme eines Applikators, einen Applikator und mindestens eine Kammer, enthaltend eine Indikatorsubstanz gewählt aus pH-Indikatoren, Redox-Indikatoren, Fluoreszenzindikatoren, Chemolumineszenz-Indikatoren, Vitalitätsindikatoren und Farbindikatoren, und eine weitere Kammer, enthaltend Puffersubstanzen, Säuren, Basen, Lösungsmittel, Salze, Enzyme, Coenzyme, Vitamine und/oder Stopp-Reagenzien, wobei die erste Kammer mit der Tasche über einen selektiv zu öffnenden Durchgangsbereich verbindbar ist und die zweite Kammer über einen selektiv zu öffnenden Durchgangsbereich mit der ersten Kammer und/oder der Tasche verbindbar ist.

9. Vorrichtung nach Anspruch 8 mit drei Kammern.

10. Vorrichtung nach einem der Ansprüche 8 oder 9, wobei alle vorhandenen Kammern derart über selektiv zu öffnende Durchgangsbereiche verbunden sind, dass alle darin enthaltenden Substanzen gerichtet seriell und/oder parallel in die Tasche überführt werden können.

11. Vorrichtung nach einem der Ansprüche 8 bis 10, wobei der Applikator ein selbstschneidendes Gewinde aufweist.

12. Vorrichtung nach einem der Ansprüche 8 bis 10, wobei auf der Vorrichtung ein Referenzsignal, vorzugsweise eine Farbskala, angebracht ist.

13. Kit zur Durchführung eines Analysenverfahrens auf Karies- und/oder Parodontitis auslösende Bakterien, umfassend eine Vorrichtung gemäß einem der Ansprüche 8 bis 12 und eine Abformmasse, enthaltend diagnostisch wirksame Zusätze.

14. Kit zur Durchführung eines Analysenverfahrens auf Karies- und/oder Parodontitis auslösende Bakterien, umfassend eine Vorrichtung gemäß einem der Ansprüche 8 bis 12 und ein Behältnis, umfassend Substanzen zur Durchführung einer Positivkontrolle zur Überprüfung der Funktionsfähigkeit des Analysenverfahrens.

15. Kit nach Anspruch 14, wobei das Behältnis mit der Vorrichtung verbunden ist oder als separater Bestandteil des Kits vorliegt.

## Claims

1. A method for analysis, comprising the following steps:
a) taking out an applicator from a device, comprising a pocket for receiving an applicator and at least one first chamber containing an indicator substance, the chamber being connectable to the pocket via a passage area to be opened selectively,
b) bringing the applicator into contact with a sample substance,
c) introducing the applicator into the device, preferably into the pocket,
d) activating the device by exertion of pressure on at least one chamber or by pushing the applicator into a chamber, whereby the applicator is brought into contact with an indicator substance,
e) optionally taking out the applicator after a period of time which is sufficient in order to generate a detectable signal,
f) optionally bringing the applicator into contact with a stop reagent in order to end the generation of the detectable signal after a period of time, the stop reagent being situated in a further chamber,
g) comparing the detectable signal with a reference signal,
h) optionally carrying out a positive control to check the functionality of the method for analysis,
wherein the applicator may be situated in the pocket from the beginning or is inlayed in a closed container.

2. The method as claimed in claim 1, wherein after step c) the applicator is firstly being brought into contact with a nutrient solution in a step c').

3. The method as claimed in any one of the above claims, wherein the indicator substance is being activated by mixing the indicator substance with an activating substance before activating the device according to step d).

4. The method as claimed in any one of the above claims, wherein the device is being opened by at least partial removal or pulling-up of a film or of a film constituent before step a).

5. The method as claimed in any one of the above claims, wherein the sample substance is saliva and, following step g), an impression of the tissue which is rinsed by the saliva is performed using an impression compound, containing diagnostically active additives.

6. The use of a device, comprising a pocket for receiving an applicator and at least one chamber for receiving an indicator substance and a further chamber for receiving buffer substances, acids, bases, solvents, salts, enzymes, coenzymes, vitamins and/or stop reagents, wherein the chamber is connectable to the pocket via a passage area to be opened selectively, for carrying out the method as claimed in any one of claims 1 to 5.

7. The use of a device according to claim 6 for carrying out an analysis of saliva for the detection of caries-causing bacteria, and/or for the analysis of gum pocket fluid, in particular for the detection of parodontitis-causing bacteria.

8. A device, comprising a pocket for receiving an applicator, an applicator and at least one chamber, containing an indicator substance chosen from pH indicators, redox indicators, fluorescence indicators, chemoluminescence indicators, vitality indicators and color indicators, and a further chamber, containing buffer substances, acids, bases, solvents, salts, enzymes, coenzymes, vitamins and/or stop reagents, wherein the first chamber is connectable to the pocket via a passage area to be opened selectively and the second chamber is connectable to the first chamber and/or to the pocket via a passage area to be opened selectively.

9. The device as claimed in claim 8 having three chambers.

10. The device as claimed in any one of claims 8 or 9, wherein all chambers present are connected via passage areas to be opened selectively in such a way that all substances contained therein can be transferred in a directed serial manner and/or in parallel to the pocket.

11. The device as claimed in any one of claims 8 to 10, wherein the applicator has a self-tapping thread.

12. The device as claimed in any one of claims 8 to 10, wherein a reference signal, preferably a color scale, is attached to the device.

13. A kit for carrying out a method for analysis on caries- and/or parodontitis-causing bacteria, comprising a device as claimed in any one of claims 8 to 12 and an impression compound, containing diagnostically active additives.

14. A kit for carrying out a method for analysis on caries- and/or parodontitis-causing bacteria, comprising a device as claimed in any one of claims 8 to 12 and a container, comprising substances for carrying out a positive control for checking the functionality of the method for analysis.

15. The kit as claimed in claim 14, wherein the container is connected to the device or is present as a separate constituent of the kit.

## Revendications

1. Procédé d'analyse comprenant les étapes suivantes:
a) on retire un applicateur d'un dispositif comprenant une poche destinée à recevoir un applicateur et au moins une première chambre contenant une substance indicatrice, la chambre pouvant être reliée à la poche par une zone de passage à ouverture sélective,
b) on met l'applicateur en contact avec une substance échantillon,
c) on introduit l'applicateur dans le dispositif, de préférence dans la poche,
d) on active le dispositif en exerçant une pression sur au moins une chambre ou en insérant l'applicateur dans une chambre, grâce à quoi l'applicateur est mis en contact avec une substance indicatrice,
e) on retire éventuellement l'applicateur après un intervalle de temps suffisant pour la production d'un signal décelable,
f) on met éventuellement l'applicateur en contact avec un réactif d'arrêt pour terminer la production du signal décelable après un intervalle de temps, le réactif d'arrêt se trouvant dans une autre chambre,
g) on compare le signal décelable avec un signal de référence,
h) on effectue éventuellement un contrôle positif pour vérifier le bon fonctionnement du procédé d'analyse,
l'applicateur pouvant se trouver dès le début dans la poche ou être placé dans un récipient fermé.

2. Procédé selon la revendication 1, dans lequel, après l'étape c), on met d'abord, dans une étape c', l'applicateur en contact avec une solution nutritive.

3. Procédé selon l'une des revendications précédentes, dans lequel, avant d'activer le dispositif selon l'étape d), on active la substance indicatrice en mélangeant la substance indicatrice avec une substance activatrice.

4. Procédé selon l'une des revendications précédentes, dans lequel, avant l'étape a), on ouvre le dispositif en retirant ou en remontant au moins partiellement une feuille ou un élément d'une feuille.

5. Procédé selon l'une des revendications précédentes, dans lequel la substance échantillon est constituée de salive et, après l'étape g), on réalise une empreinte du tissu baigné par la salive à l'aide d'une composition de moulage contenant des additifs à activité diagnostique.

6. Utilisation d'un dispositif comprenant une poche destinée à recevoir un applicateur, un applicateur, au moins une première chambre destinée à recevoir une substance indicatrice, et une autre chambre destinée à recevoir des substances tampons, des acides, des bases, des solvants, des sels, des enzymes, des coenzymes, des vitamines et/ou des réactifs d'arrêt, la chambre pouvant être reliée à la poche par une zone de passage à ouverture sélective, pour la mise en oeuvre du procédé selon l'une des revendications 1 à 5.

7. Utilisation d'un dispositif selon la revendication 6 pour la mise en oeuvre d'une analyse de salive pour la détection de bactéries cariogènes, et/ou pour l'analyse du liquide de la poche parodontale, en particulier pour la détection de bactéries déclenchant des parodontites.

8. Dispositif, comprenant une poche destinée à recevoir un applicateur, un applicateur et au moins une chambre contenant une substance indicatrice choisie parmi des indicateurs de pH, des indicateurs rédox, des indicateurs de fluorescence, des indicateurs de chimiluminescence, des indicateurs de vitalité et des indicateurs colorés, et une autre chambre contenant des substances tampons, des acides, des bases, des solvants, des sels, des enzymes, des coenzymes, des vitamines et/ou des réactifs d'arrêt, la première chambre pouvant être reliée à la poche par une zone de passage à ouverture sélective, et la deuxième chambre pouvant être reliée à la première chambre et/ou à la poche par une zone de passage à ouverture sélective.

9. Dispositif selon la revendication 8, comportant trois chambres.

10. Dispositif selon l'une des revendications 8 ou 9, dans lequel toutes les chambres présentes sont reliées par des zones de passage à ouverture sélective de façon que toutes les substances qui y sont contenues puissent être transférées en série et/ou en parallèle dans la poche.

11. Dispositif selon l'une des revendications 8 à 10, dans lequel l'applicateur présente un filetage autotaraudeur.

12. Dispositif selon l'une des revendications 8 à 10, sur lequel est appliqué un signal de référence, de préférence une échelle des couleurs.

13. Trousse pour la mise en oeuvre d'un procédé d'analyse de bactéries qui déclenchent des caries et/ou des parodontites, comprenant un dispositif selon l'une des revendications 8 à 12 et une composition de moulage contenant des additifs à activité diagnostique.

14. Trousse pour la mise en oeuvre d'un procédé d'analyse de bactéries qui déclenchent des caries et/ou des parodontites, comprenant un dispositif selon l'une des revendications 8 à 12 et un récipient contenant des substances pour la mise en oeuvre d'un contrôle positif destiné à la vérification du bon fonctionnement du procédé d'analyse.

15. Trousse selon la revendication 14, dans laquelle le récipient est relié au dispositif ou se présente sous forme d'un élément séparé de la trousse.
